# EUROPEAN PATENT APPLICATION

(11) **EP 1 099 756 A1**
(43) Date of publication of application: **16.05.2001**
(21) Application number: 99929854.0
(22) Date of filing: 15.07.1999
(51) Int. Cl.: C12N 15/10, C12N 11/00, C12Q 1/68, C07K 1/22

(54) **LABELED COMPLEX, PROCESS FOR PRODUCING THE SAME AND PROCESS FOR UTILIZING THE SAME**

(30) Priority: 22.07.1998 JP 20605798
(71) Applicant: Japan as represented by Director-General, Agency of Industrial Science and Technology, Tokyo 100-8921 (JP); Precision System Science Co., Ltd., Inagi-shi, Tokyo 206-0812 (JP); Machida, Masayuki, Tsukuba-shi, Ibaraki-ken 305-0046 (JP)
(72) Inventor: MACHIDA, Masayuki, Molecu. Bio. Dpt. Nat. Ins. of, Tsukuba-shi, Ibara (JP); TAJIMA, Hideji, Precision System Science Co., Ltd, Inagi-shi, Tokyo 206-0812 (JP)
(74) Representative: Popp, Eugen, Dr.
(86) International application number: JP9903824
(87) International publication number: WO0005357

(57) **Abstract**

The present invention relates to a labeled complex, a process for producing same and a process for utilizing same. The objects are to enable more than thousands or tens of thousands of orders of various micro particles to be discriminated as polymolecule markings in combinatorial chemistry with high sensitivity, high accuracy, consistentency, clarity, and also to satisfy each of: improvement of capture capability of targets, improvement of discrimination sensitivity; and increase of the number that can be discriminated at the same time.

The present invention comprises a micro particle, a large number of target receptors, one end of each of which is bonded with the micro particle, and labeled substances bonded with the other end of each target receptor. The target receptor attaches to or is capable of attaching to one or more than two types of targets. The configuration of the labeled substances is such that a predetermined type thereof is contained at a predetermined molar ratio, and is distributed to all target receptors bonded with the carrier.

## Description

### TECHNICAL FIELD

The present invention relates to a labeled complex, a process for producing same and a process for utilizing same. Carriers such as micro particles and the like that have a large surface area per unit weight provide an excellent solid body for attaching biological molecules thereon, and also labeling these carriers with luminescent substances such as fluorescent substances and the like enables them to be detected with high sensitivity. The present invention is to be used for examination, measurement, reaction, production, sampling, observation and the like in various areas such as genetic engineering, medical practice, medicines, physiological sanitation, health care, organisms, food, materials and the like in the fields of agricultural science, pharmaceutical science, medical science, physical sciences such as chemistry, biology and the like, and engineering science.

### BACKGROUND ART

Heretofore, to label micro particles for discrimination with high sensitivity, luminescent substances such as fluorescent substances and the like have been used. For example, to measure nucleic acid in a sample, by using a DNA probe that is bonded with fluorescent micro particles, and a large sized non fluorescent micro particle that is bonded with a different DNA probe, hybridization is performed with a DNA probe in the sample to form double strands. Subsequently, by using a filter of the mean size of the sizes of the two types of micro particle, unbound redundant fluorescent particles are removed, reacted with a restriction enzyme, double strand portions are cut, isolated fluorescent micro particles are separated by the filter and fed into a flow cell, and particles are discriminated by fluorescence in order to count the number of particles, and hence the density of the DNA probe in the sample is measured automatically (Publication of Japanese Unexamined Patent Application No. Hei 6-343496).

Furthermore, there is a method in which by using a fluorescent micro particle P with a fluorescent wavelength X, which is bonded with a substance to be bonded to a part A, being a part with specificity in a test agent, and a fluorescent micro particle Q with a fluorescent wavelength Y, which is bonded with a substance to be bonded to a part B, being a part with different specificity from the abovementioned part A in the test agent, the fluorescent micro particle P and the fluorescent micro particle Q are both specifically bonded in the test agent, a certain quantity of the liquid mixture obtained is poured into a flow cytometer, and by measuring the two types of fluorescence containing forward-scattered light emitted from the fluorescent micro particles P, Q, and the fluorescence wavelengths X, Y in the abovementioned test reagent respectively, latex fixation by reaction with a ligand is discriminated for measurement by a particle measurement device (Publication of Japanese Unexamined Patent Application No. Hei 5-107249).

As described above, heretofore not more than one type of fluorescent substance has been attached to each particle, or the fluorescent substance itself has been formed like a micro particle, to which bonding material is attached. On the other hand, types of fluorescent substances that enable discrimination with high sensitivity, having no harmful effect on biological compounds such as DNA and the like, are limited, having not more than several tens of types. Therefore, with conventional methods, no more than several tens of types of discrimination can be realized. For example, thousands or tens of thousands of types of specified substances cannot be discriminated by fluorescent substances only.

On the other hand, there is a method wherein, to enable the discrimination of many types of objects by a plurality of types of fluorescent substances, these fluorescent substances are bonded and attached to micro particles to detect antigens and/or antibodies (Publication of Japanese Unexamined Patent Application No. Sho 60-35265). However, high-density attachment of many fluorescent substances on a small region like a micro particle, which causes energy shifts and quenching (extinction) between fluorescent substances, has a harmful effect on the discrimination of spectrum and fluorescence intensity. Therefore, there is a problem in that it is not possible to label the micro particles to discriminate more than thousands or tens of thousands of types of objects with fluorescent substances. Furthermore, there is another problem in that, increasing the emission intensity and the discriminated number by increasing the number of fluorescent substances attached to the surface of the micro particle, and increasing the area for holding information in order to increase the capacity to capture object substances on the micro particle surface in order to increase the amount of information that the micro particle surface holds, conflict with each other so that it is not possible to satisfy both abilities.

### DISCLOSURE OF THE INVENTION

Accordingly, the present invention firstly provides a labeled complex, a process for producing same and a process for utilizing same, that enables the discrimination of more than hundreds, thousands and tens of thousands of types of micro objects for polymolecule marking in combinatorial chemistry; with high sensitivity, high accuracy, stability and clarity.

Secondly the invention provides an excellent labeled complex, a process for producing same and a process for utilizing same, that can satisfy the development of the capture capacity and information maintaining capacity of substances, and development of the ability of identification such as identification sense, identifying number and the like at the same time.

Thirdly the invention provides a labeled complex, a process for producing same and a process for utilizing same, wherein, by using carriers like micro particles and the like, a large amount of information per unit weight and unit quantity are labeled, so that the usage of many labeled containers is avoided, and hence space is saved and equipment scale is reduced. Furthermore, by simultaneously adding a process in the same condition, a highly accurate, effective and rapid process can be performed.

Fourthly the invention provides a labeled complex, a process for producing same and a process for utilizing same, wherein by labeling carriers such as magnetic particles and the like, automation of various types of processes can be realized on a continuum from dispensation to measurement, so that the function of magnetic particles can be rapidly increased.

Fifthly the invention provides a labeled complex, a process for producing same and a process for utilizing same, wherein the production can be performed easily, with low cost and consistent quality, and also the discrimination and measurement are easy with high accuracy and reliability.

Sixthly the invention provides a labeled complex, a process for producing same and a process for utilizing same, wherein general use and variable utilization is possible.

Seventhly the invention provides a labeled complex, a process for producing same and a process for utilizing same, which enables fine labeling for ascertaining the relationship between transcriptional control factors that can perform transcription control for substances such as various proteins and the like, and various base sequences, and corresponding substances such as DNA fragments and the like which have different base sequences, and hence it is possible to contribute significantly to research into the development of cancers and organisms, the manufacture of medicines and chemicals, gene technology, and the like.

To solve the above technical problems, a first aspect of the invention comprises a carrier, a large number of target receptors bonded with the carrier, and labeled substances bonded with each target receptor at different locations from the location at which the carrier is bonded. The target receptor holds or can hold one or two or more types of targets, and in all of the labeled substances, predetermined types are contained at predetermined molar ratios.

Here, a "carrier" can hold a specific substance, or a small object that is suspended in a liquid for use, which includes, for example, particulate, fibrous or gel substances (the form is not critical). Furthermore, macromolecule substances that are soluble in a solvent may be included. There is no particular restriction in the form, and for example, it may be spherical. The particle size is not expressly defined, however, it is preferably of the order of about 0.1µm ∼ about 1mm. It is preferable that its micro particles are formed by, for example, synthetic resin beads such as polystyrene, nylon and the like, latex particles, glass beads, gel substances, or metallic particles such as magnetic particles and the like.

The "labeled substance" is not limited to luminescent substances, which require excitation light, such as luminescence, phosphorescence and the like, but chemiluminescence or bioluminescence, which do not require excitation light may be used. In addition, for example, a substance that reacts to a specific substance specifically, a radioactive substance such as a radioisotope and the like, or a substance that emits electromagnetic radiation, such as an infrared ray and the like, may be used. Furthermore, it is possible to use a combination of these. Here, it is preferable that all of the labeled substances for one carrier are distributed for bonding with all or almost all of the target receptors bonded with the one carrier. This is because with the distributed labeled substances being separated, quenching can be prevented, and also the production process is simple. The method for distributing to each target receptor involves for example, one of every molecule of the labeled substances, an equal quantity of each molecule of the labeled substances, an equal quantity of the labeled substances, or one of every type of molecule of the labeled substances or the like.

The following is the reason why labeled substances and target receptors are not bonded separately with a carrier such as a micro particle and the like, but target receptors bonded with labeled substances are bonded with the carrier.
(1) There is a possibility in that if, to discriminate more than thousands or tens of thousands of types, a large quantity of many types of labeled substances are directly bonded with the limited area of a carrier, the region for bonding or holding diagnostic substances or targets is reduced, and the capacity to hold and capture is degraded. However, if a labeled substance is bonded with a target receptor itself, it is possible to achieve an improvement in both of the abilities of hold-capture capacity and discrimination capacity of targets and diagnostic substances.
(2) By bonding a labeled substance with a target receptor, compared with the case where the labeled substance is attached directly to a carrier such as a micro particle and the like, the space and distance between labeled substances are expanded, energy movement between the labeled substances and occurrence of quenching are prevented, and the possibility of discrimination by stable emission and the like can be guaranteed more reliably even if a lot of labeled substances are held by the carrier.
(3) Being held by a carrier such as a micro particle and the like, suspension and distribution in a liquid can be take place uniformly and equally. Therefore, statistical error is reduced, and hence it is possible to perform the process with high reliability and accuracy.

A target receptor is fixed to a carrier such as a micro particle and the like, for example, by utilizing bonding including physical or chemical bonding such as attachment, adsorption, and adhesion through the many holes, gaps, or unevenness, which the carrier has, or a specific interaction of biotin, avidin and the like.

A "target" can be, for example, information itself such as biological information of gene information, immune information and the like, information of the chemical compound structure and the like, or DNA and the like that has information, a chemical compound including biochemicals such as amino acid, sugar and the like, living organisms such as cells, viruses, bacteria and the like, a part of a living organism, or various other types of substances. Furthermore, "to hold a target" also includes the case where a target receptor itself is the target, or contains a target, or the target receptor holds a target by adsorption, reaction, interaction and the like.

With "greater than or equal to one type of target", for example, labeling can be performed for each group of a plurality of types of target per carrier, and hence it is possible to examine and measure the common character and the like of each target group.

The "location on which the carrier is bonded" and the "different location" may both be greater than or equal to two locations. Furthermore, the further the "part on which the carrier is bonded" and the "different part" are apart, the greater the effect of quenching prevention. Accordingly, it is the most desirable if the "part on which the carrier is bonded" is at one end of the target receptor, and the "different part" is at the other end of the target receptor. "One end" and the "other end" include the vicinities thereof.

According to the present invention, by specifying the types of labeled substances and the molar ratio as appropriate, it is possible to discriminate orders of more than hundreds, thousands, or tens of thousands of different targets by association. Furthermore, even though various labeled complexes are mixed in the same container and processed at the same time, since each target has been labeled, the result of the process can be obtained for each target. Consequently, it is not necessary to prepare a large number of containers for the discrimination of each target, and hence the operation can be carried out rapidly and effectively.

According to the present invention, as described above, on the surface of a carrier such as a micro particle and the like, an increase in the region for labeling and an increase in the region for target receptors are not in conflict with each other. On the contrary, the increase in target receptors brings about both an increase in the target holding capacity, and an increase in the quantity of labeled substances, and hence increases the discrimination capability including the discrimination number. Furthermore, by changing only the target receptors, a great variety of processes is possible for different purposes. Moreover, the present invention bonds labeled substances via target receptors, and does not bond the labeled substances directly with a carrier such as a micro particle and the like. Consequently, the space between the labeled substances can be larger than in the case where substances are bonded directly with the carrier, so that interactions such as energy movement between labeled substances and quenching (in the case where a luminescent material is used) are prevented. Therefore, it is possible to discriminate many substances consistently and with high accuracy.

A second aspect of the invention is that in the first aspect of the invention, all of the labeled substances are distributed to almost all target receptors bonded with one carrier, and one target receptor is bonded with one type of labeled substance. According to the present invention, since the number of each type of target receptor can be determined according to a molar ratio, the construction is simple, and the production process is easy.

A third aspect of the invention is that, in either one of the first aspect of the invention or the second aspect of the invention, the target receptor, which is bonded with the carrier on a part thereof, and bonded with the labeled substance on the other part thereof, is formed in a slender shape such as a line, a thread, a hair, a stick and the like.

The size of the "slender shape" is not expressly defined. However, for example, compared with the size of the micro particle, it is sufficiently narrower in size, and the length is longer than the thickness. For example, the form is as long as or sufficiently longer than the particle size, for example, about 10 times as long as the particle size, for example, about 10µm.

The reason why a slender shape is formed is to make it play a role as a spacer where, by attaching a labeled substance such as a luminescent material and the like on one end, compared with the case of attaching the labeled substance to a carrier such as a micro particle and the like, the space to the carrier and the space and distance between the labeled substances are expanded, and hence energy movement between the labeled substances and the occurrence of quenching are prevented, so that it guarantees more reliably the possibility of consistent discrimination of emissions and the like. According to the present invention, since a larger space can be obtained between the labeled substances compared with direct bonding to the carrier, interactions such as energy movement between the labeled substances, quenching (in the case of luminescent material), and the like are prevented, so that for example a number of substances, more than thousands and tens of thousands, can be discriminated consistently and with high accuracy.

A fourth aspect of the invention is that in any one of the first aspect of the invention through to the third aspect of the invention, the aforementioned target receptors are, for example, chemical compounds which contain biopolymers such as nucleic acids, peptides, proteins, polysaccharides, lipids and the like, or living beings such as viruses, bacteria and the like or a part thereof, or substances which hold or are able to hold them. According to the present invention, nucleic acids or substances other than nucleic acid can be used as the target receptors. For example, in the case where a nucleic acid is used, among various proteins, transcription control elements, which can control transcription of various base sequences, or DNA fragments having different base sequences can be found rapidly and easily by labeling many proteins or substances such as base sequences and the like. In this manner, it is possible to contribute significantly to research into the development of cancers and organisms. In addition, by using various substances, the relation between various substances, for example, the intensity of interaction, similarity of structure, and the like can be identified.

Here, "nucleic acid" is deoxyribonucleic acid (DNA) or ribonucleic acid .(RNA) in the narrow definition, and may include PNA (Peptide Nucleic Acid) in a wider definition. RNA includes mRNA, tRNA and rRNA. Furthermore, not only whole DNA and RNA, but also the case of fragments of DNA and RNA is included. Here, antigens and antibodies are also included in proteins, polysaccharides, lipids and the like.

A fifth aspect of the invention is that in any one of the first aspect of invention through to the fourth aspect of the invention, the target receptors comprise nucleic acids having a predetermined double strand base sequence, the labeled substance is bonded with only a single strand at one location, and the carrier is bonded with the other single strand in at another location. It is preferable that the location at which the labeled substance is bonded and the location at which the carrier is bonded are as far apart as possible. Preferably the locations correspond to one end and the other end of the double strand. Here, for example, in the case where fluorescent substances are used as the labeled substances, one method of bonding the fluorescent substance with DNA or RNA is, for example, by covalent bonds such as thiocarbamide bonds and the like. With the present invention, double strand nucleic acid can be discriminated distinctly by labeled substances.

A sixth aspect of the invention is that in any one of the first aspect of the invention through to the fifth aspect of the invention, the target receptor comprises nucleic acid having a predetermined double strand base sequence, the labeled substance is bonded only with one location of a single strand, and the carrier is fixed to another location of the single strand. With the present invention, by denaturing the target receptor to a single strand, the base sequence combination in which a relation such as complementation, homology and the like is high can be ascertained rapidly and reliably by hybridization.

A seventh aspect of the invention is that in any one of the first aspect of the invention through to the fifth aspect of the invention, the target receptor is a single strand nucleic acid. Here, it is possible to obtain a single strand, for example by heat treatment or denaturing, being the addition of an alkaline solution and the like. With the present invention, by denaturing the target receptor to a single strand, the base sequence combination in which a relation such as complementation, homology and the like is high can be ascertained rapidly and reliably by hybridization.

An eighth aspect of the invention is that in any one of the first aspect of the invention through to the fifth aspect of the invention, the labeled substance is a fluorescent substance, mineral phosphate, or luminescent substance such as a chemiluminescent substance and the like.

Here, the fluorescent substance is, for example, an organic substance such as FITC (fluorescein isothiocyanate), rhodamine, isothiocyanate, IRF40, an organic substance such as CY3, CY5 and the like, or an inorganic substance such as europium complex and the like which emits long-lived fluorescence. The present invention uses the luminescence of luminescent material and the like such as fluorescence, phosphorescence and the like as labeled substances. In this manner, the discrimination capability of targets is increased, and also stable and high reliability can be obtained.

A ninth aspect of the invention is that in any one of the first aspect of the invention through to the eighth aspect of the invention, the type of the luminescent substance can be discriminated by any one of the excitation wavelength, emission wavelength, emission intensity; degree of emission polarization, emission phase or emission lifetime. Here, "degree of emission polarization" is a method for measuring how much the fluorescence generated by exciting the fluorescent molecules by polarized excitation light is polarized. With the present invention, differences in various characteristics of luminescent materials are utilized, enabling the labeling of many types.

A tenth of aspect of the invention is that in the ninth aspect of the invention, the carrier is coated with one of a pair of chemical compounds that are specifically bonded, such as avidin, biotin and the like, the target receptor is a DNA fragment having a predetermined base sequence, the other chemical compound of the chemical compound pair is bonded with one end thereof, and the fluorescent substance is bonded with the other end. Here, the "pair of chemical compounds that are specifically bonded", could be a pair of avidin and biotin, or further, a pair of antigen and antibody such as DIG (digoxin-genin) and anti-DIG antibody, and the like. With the present invention, the target receptor can be fixed to the carrier with low cost, easily and reliably.

An eleventh aspect of the invention is that in any one of the first aspect of the invention through to the tenth aspect of the invention, the carrier has objects of action at a distance such as magnetic particles and the like, which can be controlled remotely.

Here, the objects of action at a distance could be magnetic particles, charged particles, dielectrics, chemotactic microorganisms and the like. In the case where magnetic particles are used, the operation can be automated, for example by a pipette device wherein a magnetic body is built-in. In the case where charged particles are used for the carrier, if the same charge is provided with the labeled substances, these repel each other so that the labeled substances can be separated from one another. Especially, in the case where the carrier is formed by magnetic particles, by using a device that exerts a magnetic force inside the liquid path of a pipette device, processes such as dispensing, movement, suspension, agitation, separation, washing, recovery, resuspension and the like can be completely automated from the start of the process to the measurement.

A twelfth aspect of the invention is that in a process for producing a labeled complex according to any one of the first aspect of the invention through to the eleventh aspect of the invention, the process has a step for forming target receptors, which are bonded with labeled substances, and hold or are capable of holding specific targets, and a step for bonding the target receptors with the carrier. The order of the two processes is not critical, and they may be performed at the same time. With the present invention, if a large number of labeled substances are formed, for example, by microscopically equal amounts or equal molecule amounts, and then suspension, mixing and the like are performed with many target receptors, it is possible to form target receptors bonded with labeled substances easily and uniformly. Similarly, by performing suspension, mixing and the like of many target receptors and many carriers, it is possible to produce many homogeneous labeled complexes with a low statistical error. Furthermore, with a labeled complex produced by the present invention, the increase of the region for labeling and the increase of the region for target receptors on the surface of carriers are not in conflict with each other on the limited surface. On the contrary, the increase of target receptors brings about both an increase in the target holding capacity, and an increase in the number of labeled substances, and hence increases the discrimination capability including the discrimination number. Moreover, by changing only the target receptors, a great number of general-purpose processes is possible. Furthermore, with a labeled complex produced by the present invention, labeled substances are bonded via target receptors, and the labeled substances are not directly bonded with a carrier. Consequently, the space between the labeled substances can be larger than if the substances are bonded directly with the carrier, so that interactions such as energy movement between labeled substances and quenching (in the case where a luminescent material is used) is prevented. Therefore, it is possible to discriminate many substances consistently and with high accuracy.

A thirteenth aspect of the invention is that in the twelfth aspect of the invention, the step for bonding target receptors with the carriers is performed by mixing the carriers with which the target receptors are to be bonded, in a liquid wherein a large number of target receptors which are bonded with labeled substances are suspended such that all of the labeled substances are of the types and the molar ratios that are determined according to the types of the target receptors or the types and the quantity ratios of the target receptors. Here, "a large number" needs to be a number such that statistical errors can be ignored. The "molar ratio" needs to be determined accurately such that the ratio is of an order high enough to ignore statistical errors. Furthermore, "suspension" is preferably to suspend such that the target receptors become sufficiently homogeneous.

With the present invention, the arrangement is such that carriers are mixed in a liquid in which a large number of target receptors bonded with labeled substances are suspended such that specific types of labeled substances have a specific molar ratio. The carriers contact and are bonded with a part of the target receptors randomly selected among the large number of target receptors. Consequently, the types and molar ratio of the labeled substances that the target receptors that contact and are bonded with the carriers have, reflect the types and the molar ratio of the labeled substances that all of the target receptors suspended have, within the statistical error, and the target receptors are selected at random. Hence it is possible to produce a labeled complex having labeled substances that reflect the intended types and molar ratio faithfully within the statistical error, easily and with high accuracy.

Here, the process for generating target receptors bonded with labeled substances can also be performed by mixing a large number of the target receptors, with the liquid within which the labeled substances are suspended such that all of the labeled substances are of types and molar ratios that are determined according to the types of the target receptors to be bonded or the types and the quantity ratios of the target receptors to be bonded.

A fourteenth aspect of the invention is that in the twelfth aspect of the invention, the step for generating the target receptors has a step for synthesizing a single strand nucleic acid that is bonded with a labeled substance, and that has a predetermined base sequence, and synthesizing another single strand nucleic acid that has a high relation with the base sequence, and that is processed to be capable of being bonded with the carriers, and generating a double strand nucleic acid by annealing these. Here, "high relation" includes a case of high complementation or homology, and a case where interaction is strong (high affinity). With the present invention, various target receptors can be generated easily and with low cost.

A fifteenth aspect of the invention is that in the thirteenth aspect of the invention, the step for generating the target receptors has a step wherein, by using a first primer for reproduction of a single strand nucleic acid that is bonded with the labeled substances and that has a predetermined base sequence, and a second primer for reproduction of the other single strand nucleic acid to be bonded with the carrier, a double strand nucleic acid is synthesized and amplified. With the sixteenth aspect of the invention, a large amount of labeled complex can be produced easily and rapidly depending on the target receptors.

A sixteenth aspect of the invention is that in the twelfth aspect of the invention, the step for bonding the target receptors with carriers, or the step for generating the target receptors, synthesizes and amplifies double strand nucleic acid by using a first primer for reproduction of a single strand nucleic acid with a predetermined base sequence, which combines with either one of the labeled substance and the carrier, and also provides a restriction enzyme process at the opposite end to the end that is bonded with the labeled substance or a carrier and, via an adapter composed of DNA ligase and the like, bonds a carrier or a labeled substance onto the single strand side to combine the target receptor with the carrier, or generates a target reservoir. With the present invention, it is possible to easily produce a large quantity of labeled complex for determining a base sequence that has a high relation of complementation, homology or the like with the base sequence that is the target of labeling.

A seventeenth aspect of the invention is that in the sixteenth aspect of the invention, the step for generating the target receptor includes a step for removing a single strand that is not bonded with the labeled substance or the carrier by denaturation. With the present invention, it is possible to easily produce a large quantity of labeled complex for determining a base sequence that has a high relation of complementation, homology or the like with the base sequence that is the target of labeling.

An eighteenth aspect of the invention is that in the twelfth aspect of the invention, the step for bonding the target receptor with the carrier bonds the target receptor and the carrier by utilizing bonding including physical or chemical bonding such as attachment, adsorption, adhesion through the many holes, gaps, or irregularities that the carrier has, or a specific interaction of biotin, avidin and the like, to suspend the target receptor and the carrier. In this manner, various types of labeled complexes can be obtained, thus enabling the utilization for various treatments.

A nineteenth aspect of the invention is that in the twelfth aspect of the invention, the step for generating the target receptor is a step for generating a plurality of target receptors with which is bonded one of a pair of chemical compounds, one part of which is bonded with the labeled substance, and the other part of which is specifically bonded. The step for bonding the target receptor with the carrier is a step for bonding the target receptor with the carrier by suspending in liquid the carrier on which the other of the pair of chemical compounds is coated, and the target receptor with which the labeled substance is bonded.

A twentieth aspect of the invention has; a step for selecting a labeled complex whose type is targeted from among the labeled complex group having a large number of a plurality of types of labeled complexes wherein the types or molar ratio of the targets of the labeled complex according to any one of the first aspect of the invention through to the eleventh aspect of the invention, and the labeled substances assigned to the targets are different from each other, and a step wherein the selected labeled complex discriminates the labeled target.

With the present invention, by specifying various types and molar ratio of labeled substances, it is possible to discriminate more than hundreds, thousands, tens of thousands of orders of various targets by association. With the present invention, even though various labeled complexes are mixed in the same container and processed at the same time, since each target has been labeled, a result of the process can be obtained for each target. Consequently, it is not necessary to prepare a large number of containers for the discrimination of each target, and hence the performance can be carried out rapidly and effectively.

A twenty-first aspect of the invention is that in the twentieth aspect of the invention, the selection step has a liquid path and a magnetic device which is capable of applying magnetization to inside of the liquid path. The process is performed for the labeled complex, or the labeled complex and selective substances, using a pipette device for suction and discharge, and by operations such as quantification, isolation, apportioning, dispensing, clarity, suspension, agitation, concentration, dilution, mixing, contact, holding, capture, washing, denaturation, incubation, temperature control, extraction, recovery, transport, and the like, or by a combination of these operations.

Here, the pipette device is not limited to only a single unit, but it may be a piece of equipment within which a plurality of points, liquid paths, and storage sections are integrated. Furthermore, the liquid path may connect the point and the storage section in the pipette device containing the point and the storage section. Suction and discharge of liquid is performed by a pressure control device for controlling the pressure in the liquid path. With the present invention, each operation or bonding is carried out automatically, rapidly, easily, effectively and accurately.

A twenty-second aspect of the invention is that in either one of the twentieth aspect of the invention or the twenty-first aspect of the invention, the selection step has; a step for suspending the labeled complex group, a step for contacting the suspension in which the labeled complex group is suspended, and selective substances for selecting the object labeled complexes, and a step for extracting or separating the labeled complexes bonded with the selective substances. Here, "contact", is performed by, for example mixing the two, or by pouring one into the container in which the other is contained. With the present invention, it is possible to identify the bonding of a target receptor that has a high affinity with each target, or has some interaction. In this manner, relations such as complementation, homology similarity of structure, and the like can be identified, and hence it is possible to ascertain, for example, a transcription control factor and a DNA fragment having a different base sequence.

A twenty-third aspect of the invention is that in the twenty-second aspect of the invention, the selection step has a step for labeling the selective substances with different types of labeled substances from the labeled substances contained in the labeled complex, and for extracting and separating the labeled complex bonded with the selective substances based on the labeled substances. With the present invention, an object labeled complex can be selected and separated, or discriminated reliably and easily.

A twenty-fourth aspect of the invention is that in either one of the twentieth aspect of the invention or the twenty-first aspect of the invention, the discrimination step, in the case where the labeled substances are luminescent substances, computes the ratio of the amount of the type based on a result of the measurement of intensity of each emission wavelength which the labeled complex emits, to discriminate the corresponding target. With the present invention, the corresponding target can be discriminated easily and reliably.

A twenty-fifth aspect of the invention is that in either one of the twentieth aspect of the invention or the twenty-first aspect of the invention, with the selection step, the target receptor is a double strand nucleic acid with a predetermined base sequence, and in the case where the labeled substance and the carrier are bonded with only one single strand thereof, the other single strand is removed by denaturation. Furthermore, for the selective substances, nucleic acid having a predetermined base sequence is used. In this manner, hybridization is performed on the target receptors that have a structure with high complementation or homology with the base sequence for test of the selective substances. By discriminating the labeled complex that captured the labeled substances, the structure of the target is determined; With the present invention, it is possible to determine easily and effectively a base sequence that has high relation of complementation, homology, and/or the like, which hybridizes with a specific base sequence.

A twenty-sixth aspect of the invention is that in either one of the twentieth aspect of the invention and the twenty-first aspect of the invention, the selection step has a step for contacting stationary phases on which selective substances are fixed, and a liquid wherein the labeled complex group is suspended, and a step for selecting a labeled complex bonded with the selective substances on the stationary phase by removing the suspension by washing. With the present invention, since substances other than labeled complexes that are captured on the stationary phase are removed by washing for selection of the labeled complex, the selection is easy and reliable.

A twenty-seventh aspect of the invention is that in the twenty-sixth aspect of the invention, the selection step has a step wherein labeled complexes are eluted physically or chemically from the stationary phases for extraction, and are selected by separation, washing or the like. Here, "physical elution" includes cases where vibration, impact, pressure, heat and the like are added. With the present invention, labeled complexes can be discriminated reliably and easily.

A twenty-eighth aspect of the invention is that in the twenty-sixth aspect of the invention, magnetic particles are used for the stationary phases, the carriers of the labeled complex are formed by magnetic particles or non-magnetic particles and compared with the magnetic particles of the stationary phase, the magnetic particles of the labeled complex experience a smaller magnetic field for the same external magnetic field. With the present invention, since remote control operation can be utilized, the operation and process are automated, and hence the process can be performed rapidly and easily.

A twenty-ninth aspect of the invention is that in the twenty-sixth aspect of the invention, the selection substance is labeled with one of a pair of chemical compounds that are specifically bonded, being a labeled substance, and also in the selection step, a liquid wherein the conjugation of the selective substances and labeled complexes is suspended, is contacted with the stationary phase on which the other of the pair of chemical compounds is fixed, the conjugation is bonded with the stationary phase, the substances other than those bonded with the stationary phase are washed to be removed, the target receptor is denatured to be a single strand, and the labeled complex is eluted and recovered by extraction or separation. With the present invention, the complex is first captured on the stationary phase, and the captured complex is extracted for selection and separation, and hence the labeled complex can be discriminated reliably and accurately.

A thirtieth aspect of the invention is that in either one of the twentieth aspect of the invention and the twenty-first aspect of the invention, the selection step has; a step for labeling the labeled complex by luminescent substances, for labeling the selective substances by different types of luminescent substances from the labeled substances, and for mixing and contacting the liquid in which the labeled complex group is suspended with the selective substances, and a step for passing suspended liquid of the labeled complex group including labeled complexes bonded with the selective substances through a translucent narrow tube. The discrimination step has a step for receiving light when the suspended liquid of the labeled complex group passes through the narrow tube, and a step wherein, with respect to the labeled complex selected by the measurement of the intensity of light emitted by the selective substance, based on the result of a measurement of the intensity of light emitted by the labeled complex, the types and the molar ratio are computed to discriminate the corresponding target.

Here, the flow rate in the narrow tube of the suspended liquid may be adjusted such that, for example, the labeled complex passes through the inside of the narrow tube without particles overlapping. The flow rate is determined by the inner diameter of the narrow tube, the concentration of the suspended liquid and the like. With the present invention, selection of labeled complexes and discrimination of the targets can be performed at the same time by measuring the light intensity, and hence it is possible to detect the targets effectively and reliably.

A thirty-first aspect of the invention is that in the thirtieth aspect of the invention, in the case where the discrimination substances or selective substances are fluorescent substances or mineral phosphates, in the step for passing the suspended liquid through the narrow tube, an excitation light for exciting the substances is emitted toward the narrow tube. With the present invention, targets can be detected positively and with high reliability.

A thirty-second aspect of the invention has a transfer pump for transferring a liquid within which a labeled complex group is suspended, a translucent narrow tube through which the suspension passes, a light detecting device for detecting light from the discrimination substances and selective substances of the labeled complex when passing through the narrow tube, and an analyzing device for analyzing the intensity of light received by the light detecting device, selecting the labeled complex, and discriminating a target.

The transfer pump is controlled, for example, such that the flow rate allows the labeled complexes to flow without overlapping each other in the narrow tube. With the present invention, selection of a labeled complex and discrimination of targets can be performed at the same time by the measurement of the light intensity, and hence it is possible to detect the target effectively and reliably.

Furthermore, the diameter of the narrow tube is determined depending on the size of the labeled complex used, concentration, flow rate, amount of liquid and the like.

A thirty-third aspect of the invention is that in the thirty-second aspect of the invention, there is further provided an irradiating device for externally radiating excitation light toward the narrow tube for, in the case where the discrimination substances or selective substances are fluorescent substances or mineral phosphates, exciting the substances, or providing light for scattering to obtain scattered light from the substances.

A thirty-fourth aspect of the invention is that in the thirty-second aspect of the invention, the narrow tube forms part of a closed circuit, and the suspension of the labeled complex group is circulated in the closed circuit.

With the present invention, since the suspension is circulated, the measurement can be repeated, and hence the accuracy of the measurement can be enhanced.

### BRIEF DESCRIPTION OF THE DRAWINGS

FIG. 1 shows labeled composite particles according to a first embodiment of the present invention.
FIG. 2 shows an example of labeled composite particles according to the first embodiment of the present invention.
FIG. 3 shows a process for producing the labeled composite particles according to a second embodiment of the present invention.
FIG. 4 shows a process for utilizing the labeled composite particles according to a third embodiment of the present invention.
FIG. 5 shows a process for utilizing the labeled composite particles according to a fourth embodiment of the present invention.
FIG. 6 shows a process for utilizing the labeled composite particles according to a fifth embodiment of the present invention.
FIG. 7 shows a process for utilizing the labeled composite particles according to a sixth embodiment of the present invention.
FIG. 8 shows a process for utilizing the labeled composite particles according to a seventh embodiment of the present invention.
FIG. 9 shows a process for utilizing the labeled composite particles according to an eighth embodiment of the present invention.
FIG. 10 shows an apparatus for utilizing the labeled composite particles according to a tenth embodiment of the present invention.

### BEST MODE FOR CARRYING OUT THE INVENTION

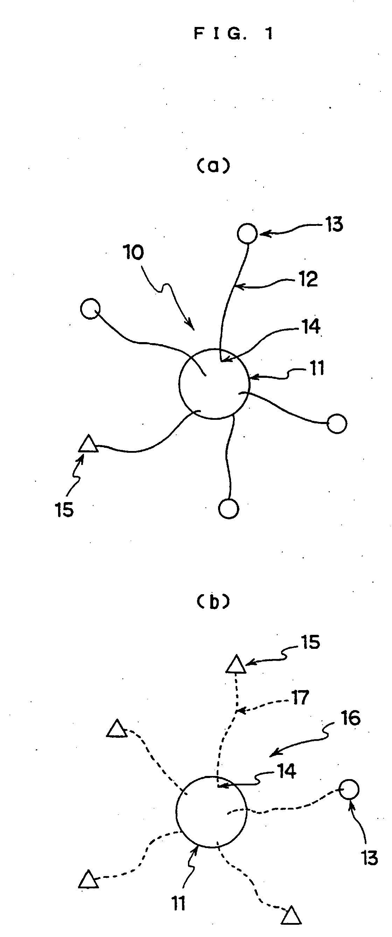

Next is a description of a labeled complex and a process for producing same, and a process for utilizing same according to embodiments of the present invention based on FIG. 1 through FIG. 10. Furthermore, the present invention is not particularly limited to these embodiments unless specified.

FIG. 1 shows a labeled composite particle 10 and a labeled composite particle 16 as a labeled complex according to a first embodiment. The labeled composite particle 10, as shown in FIG. 1 (a), has: a magnetic particle 11, being a micro particle that is one carrier; fluorescent substances 13 (○) and 15 (△), being labeled substances in which predetermined types (two types for simplicity of description) are contained at predetermined molar ratios to the whole; and multiple target receptors 12, one end 14 of which is bonded with the magnetic particle 11 by a specific bond of biotin and avidin, and is bonded with the magnetic particle 11 by utilizing bonding including chemical and physical bonding such as suction, covalent bonds and the like, and the other end of which is bonded with fluorescent substances 13, 15, and holds DNA fragments (solid lines) with a predetermined base sequence as targets. Here, the fluorescent substance 13 is, for example FITC (green), the fluorescent substance 15 is, for example rhodamine (orange) or Cy5 (Cy five, red). The fluorescent substance 13 and the fluorescent substance 15 are discriminated by excitation wavelength, emission wavelength and the like.

In FIG. 1 (a), a case where the molar ratio of the fluorescent substance 13 and the fluorescent substance 15 is, for example, 4 to 1 as a whole is illustrated with five target receptors 12 for simplicity of description. On the other hand, the labeled composite particle 16 shown in FIG. 1 (b) shows target receptors 17 which have DNA fragments (broken lines) with a different base sequence from the DNA fragment in the aforementioned target receptors 12. Here, the same symbols as in FIG. 1 (a) denote the same things. In the present embodiment, since a magnetic particle 11 is used for a micro particle, not only are the target receptors 12 held altogether, but also the labeled composite particles 11, 16 can be remotely controlled by a magnetic field. In particular, by using a pipette device that is provided with a magnetic device, various operations can be automated.

As follows is a description of how these labeled composite particles can be discriminated by labeled substances using fluorescence, with a brief example.

In the example, with regard to a labeled composite particle wherein one end of a DNA fragment having a base sequence suitable for the aforementioned target receptors is fixed onto a bead (micro particle), and two types of fluorescent substances, which are here Cy5 (red) and FITC (green) with different molar ratios, are bonded with the other ends, fluorescence intensity is measured individually, and statistical processing is performed on the measured results.

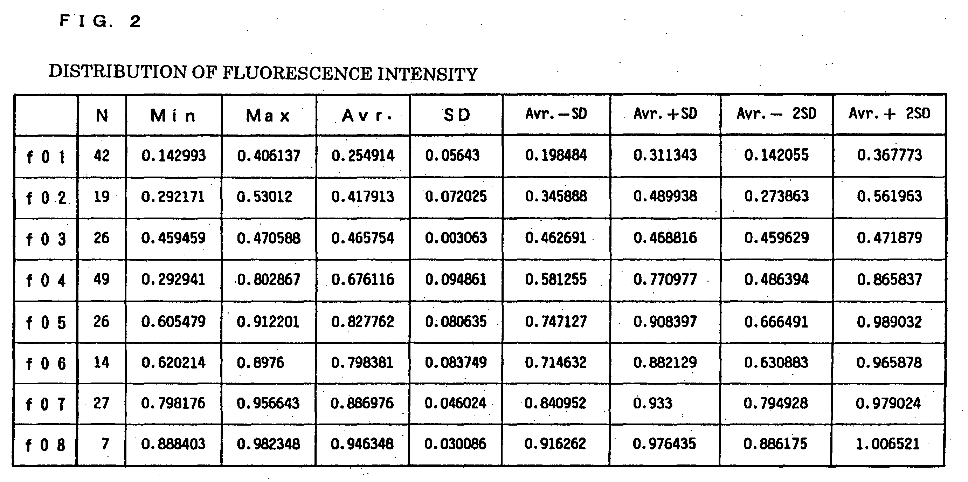

In the present example, the degree of discrimination is measured with regard to a labeled composite particle, wherein without changing the total amount of the two types of fluorescent substances, Cy5 and FITC, the total light intensity is almost constant, and only the molar ratio is changed in eight types and labeled. The method is that the fluorescence intensity of Cy5 and FITC of each individual bead is measured and numerically converted, the ratio of the fluorescence intensity of Cy5 and FITC is calculated, and the test result showing the distribution of fluorescence intensity of the beads, shown in FIG. 2, is obtained.

In FIG. 2, "N", "Min", "Max", "Avr.", "SD" show the measured number of beads, the minimum value, the maximum value, the average value, and the standard deviation respectively. Samples are prepared, wherein the total amount is fixed, and only the molar ratio is changed in eight types, in sample f01 Cy5:FITC = 0.1:0.9, sample f02 Cy5:FITC = 0.2:0.8 .... Assuming that the distribution of fluorescence intensity has a regular distribution, about 68% of beads have a fluorescence intensity ratio in the range of Avr. - SD to Avr. + SD, and about 98% are in the range of Ave. - 2SD to Avr. + 2SD. When looking at the rows of Ave. - 2SD and Avr. + 2SD in FIG. 2,
- f01: 0.14∼0.37
- f03: 0.46∼0.47
- f04: 0.49∼0.86
- f08: 0.89∼1.00
When selected in this manner, the divisions do not overlap, and hence separation and discrimination of at least these four gradations can be performed with a margin of error of less than 5%. In this experimental example, to save time, beads whose measured absolute fluorescence intensity is abnormal, or where a plurality of beads overlap, and the like are not completely removed. However, by removing these, the sharpness of separation of gradations can be further improved. Furthermore, by increasing the number of samples, statistical errors can be further reduced.

In this example, if the case of the whole amount being Cy5 is included, five gradations can be separated. Similarly, if the types of fluorescent substances are increased, and for example nine pairs of such bondings are used simply, about 200,0000 types can be discriminated.

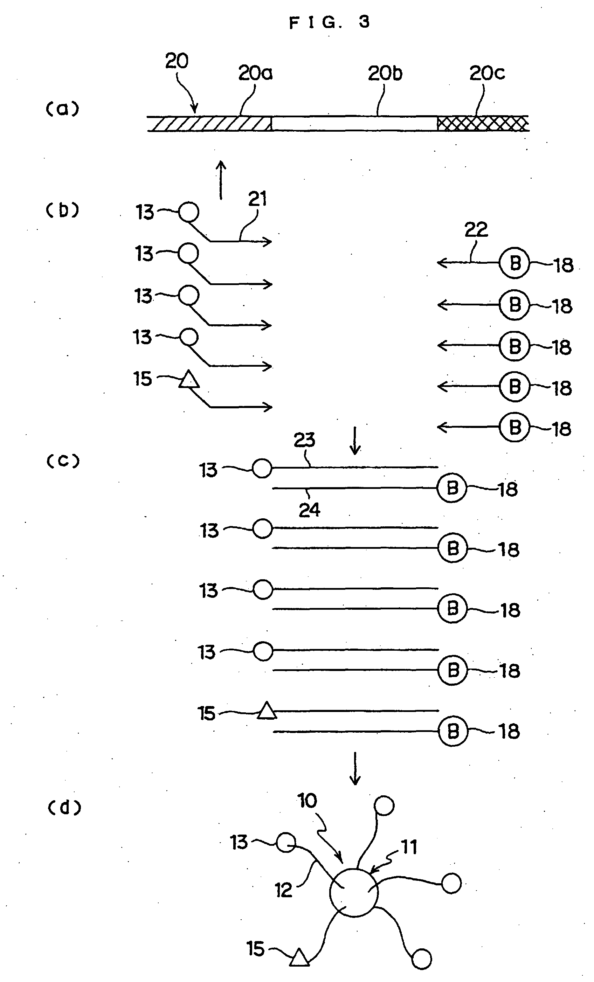

The process for producing a labeled complex according to a second embodiment will now be described based on FIG. 3.

As shown in FIG. 3, to produce the labeled composite particle 10, for example with regard to double strand DNA 20, a DNA fragment corresponding to a single strand of DNA region 20a is designated as a first primer 21, and a DNA fragment corresponding to the other single strand of DNA region 20c is designated as a second primer 22. The first primer 21 is bonded in advance with the fluorescent substance 13 or the fluorescent substance 15 by covalent bonding or the like. Furthermore, the second primer 22 is bonded in advance with biotin 18 by covalent bonding or the like. In this manner, by the PCR method (polymerase chain reaction method), by repeating a DNA synthesis reaction with the two types of primers 21 and 22 by DNA synthesis enzyme (polymerase), it is possible to limitlessly amplify the whole of DNA fragment 20, having a fluorescent substance at one end and a biotin at the other end. At this time, by using the first primer that is bonded with the fluorescent substance 13 and the fluorescent substance 15 at a ratio of 4 to 1 by quantity, target receptors, wherein a fluorescent substance 13 is bonded with a single strand 23 of the double strand DNA fragment 20 as shown in FIG. 2 (c), and the biotin 18 is bonded with the other single strand 24, and target receptors, wherein a fluorescent substance 15 is bonded with a single strand 23, and the biotin 18 is bonded with the other single strand 24 can be obtained at a ratio of 4 to 1 by quantity.

Mixture, suspension and/or agitation, are performed by dispensing, a solution in which a plurality of micro particles (beads) 11 formed by magnetic particles are suspended, or only micro particles 11, for example by using a pipette device that is provided with a liquid path and a magnetic field that is near the liquid path and separable, in a solution in which an order of more than tens of thousands of target receptors 12 obtained in this manner are suspended.

The surface of the micro particle 11 is coated in advance with avidin that is specifically bonded with the biotin 18 by a specific reaction. Then, by mixing, suspending or agitating the micro particle 11 in the suspension of the target receptors 12, one end of the target receptor 12 with which the biotin 18 is bonded reacts, is bonded and is stabilized with the surface of each micro particle 11. At that time, since the number of target receptors 12 is large, the ratio of the amount of the fluorescent substances 13 and the fluorescent substances 15 which are bonded with the other ends of the target receptors 12 bonded with the micro particles 11 reflects the ratio of the amount in the suspension almost exactly, which is a bonding in the ratio of 4 to 1 within a constant statistical error. These statistical errors converge to such a degree that they can almost be ignored, as the number of target receptors 12 that are suspended homogenously and constantly is increased.

The number of target receptors 12 to be bonded with the micro particles 11 can be controlled by changing the number of target receptors 12 or conditions of reaction time and the like. By increasing the number within the range where a major influence by energy movement or quenching does not occur among the fluorescent substances, emission intensity and capture ability can be enhanced.

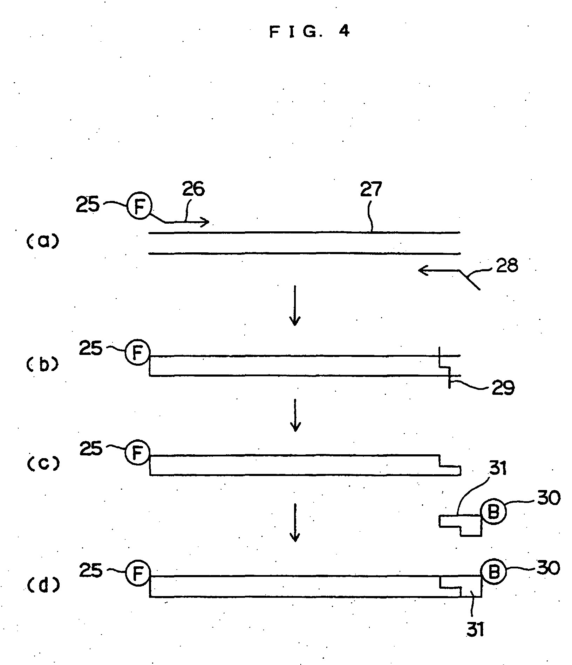

Next is a description of the process for producing a labeled complex according to a third embodiment based on FIG. 4.

As shown in FIG. 4 (a), by using the primer 26 bonded with the fluorescent substance 25, and the primer 28, which has a recognition sequence 29 of a restriction enzyme at the end, a target receptor 27 is duplicated and amplified by the aforementioned PCR. A DNA fragment 27 is, for example amplified to more than hundreds of thousands by a DNA synthesis enzyme, by using the aforementioned double strand mold DNA fragment 27, and the primers 26 and 28.

As shown in FIG. 4 (b), a restriction enzyme is added to the double strand DNA fragments obtained in this manner. The restriction enzyme recognizes the aforementioned cutting capable sequence 29, and cuts off the DNA fragment.

In FIG. 4(c), a biotin 30 bonded with an adapter 31 that is made of DNA ligase capable of being bonded with the end part that was cut out and processed, is added into the suspension of the DNA fragments. At this time, the biotin 30 is bonded with the adapter 31 such that it is bonded on the single strand side where the aforementioned fluorescent substance is bonded. In FIG. 4 (d), the biotin 30 is bonded with the target receptor that was cut out and processed. In this manner, a target receptor where the fluorescent substance 25 and the biotin 30 are bonded with only one side of a single strand, can be obtained. Here, since the process where the target receptor is bonded with the micro particle to stabilize is the same as in the process that has already been described, the description is omitted.

Next is a description of the process of producing a labeled complex according to a fourth embodiment.

Here, a labeled composite particle is used to identify the base sequence of a DNA fragment that interacts with a protein 32.

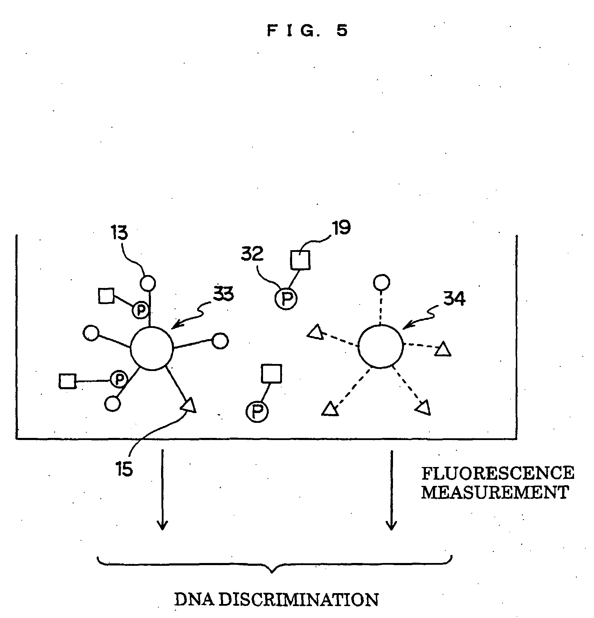

As shown in FIG. 5, a plurality of types of labeled composite particle groups is prepared. Here, for simplicity, two types of labeled composites 33, 34 are used. The target receptors of the labeled composite particle 33 and the labeled composite particle 34 are different, and are formed by DNA fragments with different base sequences. One end of the target receptors of the two particles, 33, 34 is fixed to each of the micro particles. However, the other end thereof is bonded with two types of fluorescent substances 13(○), 15 (△) in a molar ratio of 4 to 1 for the labeled composite particle 33, and in a different molar ratio of 1 to 4 for the labeled composite particle 34. The base sequences which the labeled composite particles 33, 34 have are labeled through the difference in the molar ratio. Firstly, the suspension within which the labeled composite particles 33, 34 are mixed is held in a container. The protein 32 is added to the container, being a selective substance labeled by a fluorescent substance 19(□) which is different from the fluorescent substances 13 (○), 15 (△), being labeled substances that had labeled the labeled composite particles 33, 34. By tracing the fluorescent substance 19 after a fixed period, it can be checked whether the protein 32 has become attached to one of the labeled composite particles 33, 34 or has not become attached. In the case where the protein 32 is attached to neither of the labeled composite particles 33 or 34, it is determined that there is no or an extremely small relation between the protein 32 and the base sequence of the DNA fragment.

On the other hand, in the case where the protein 32 is determined as being attached to either one of the labeled composite particles 33, 34, the labeled composite particle (33) to be bonded with the protein 32 is selected and separated. By measuring the emission intensity of the fluorescent substances 13 (○), 15 (△) of the selected and separated labeled composite particle (33), the molar ratio is computed, and hence the labeled base sequence can be discriminated from the labeled composite particle 33 that has bonded to the protein 32. As a result, a base sequence with a high affinity or high relation, which can be bonded with protein 32 is specified.

In the case where it is determined that the protein 32 is attached to both of the labeled composite particles 33, 34, with regard to the degree of relation such as affinity and the like, whether the relation such as the degree of affinity and the like is high or low can also be judged by comparing the amount of the protein 32 that the labeled composite particles 33 and 34 have attached, or the quantity ratio of the labeled composite particles 33 and 34 that have attached to the protein 32.

In the present embodiment, the arrangement may be such that the target receptors are formed by a plurality of types of proteins and the like, instead of being formed by a plurality of types of DNA fragments, and in a suspension of the labeled composite particles, DNA fragments with a specific base sequence, which are labeled by a different type of labeled substances from the labeled substances used for the labeled composite particles, are mixed as selective substances. In this case, conversely, protein with a high affinity for DNA fragments with the base sequence can be specified.

The present embodiment can be applied not only between proteins and DNA fragments, but also between two DNA fragments. In this manner, structures with high complementation and homology among base sequences of DNA fragments, and relations such as intensity of interaction and the like can be determined. Here, with the present invention, by using a magnetic particle for each labeled composite particle, and by using a pipette device that is provided with a magnetic device, or an apparatus wherein a plurality of these pipette devices is integrated, operations such as suspension, agitation, separation, transfer and the like can be automated.

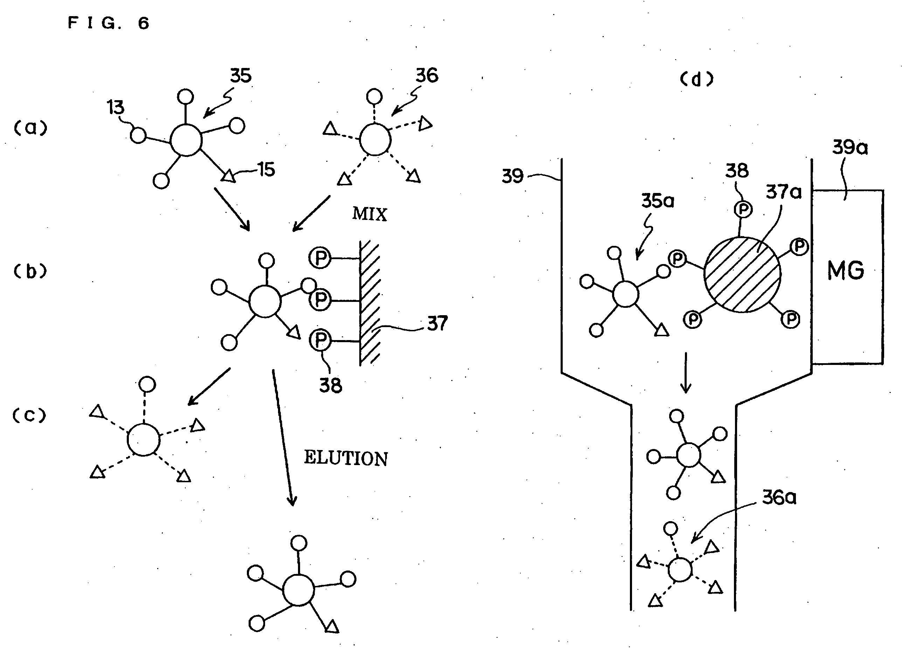

The process for utilizing a labeled complex according to a fifth embodiment will now be described based on FIG. 6.

As shown in FIG. 6 (a), a plurality of types of labeled composite particles, for example, two types of labeled composite particles 35, 36, is prepared. The target receptors of the labeled composite particle 35 and the labeled composite particle 36 are different, and are formed by DNA fragments with different base sequences. One end of the target receptors of the two particles 35, 36 is fixed to each of the micro particles. However, the other end thereof is bonded with two types of fluorescent substances 13(○), 15 (△) in a molar ratio of 4 to 1 for the labeled composite particle 34, and in a molar ratio of 1 to 4 for the labeled composite particle 36. To contact with a matrix (stationary phase) 37 on which a DNA binding protein 38 is fixed as a selective substance, the suspension within which those two types of labeled composite particles 35, 36 are mixed in is dispensed into a container in which the matrix 37 is provided.

Then, the labeled composite particle 35, which has a target receptor of a DNA fragment with a base sequence that is bonded with the DNA protein 38 by a specific reaction, is bonded with the protein 38, and captured by the matrix 37. Then after the labeled composite particles 35, 36 that have not been captured by the matrix 37 are washed, the captured labeled composite particle 35 is dissolved out by a saline solution, and hence the corresponding labeled composite particle 35 is selected, and by observing the fluorescence spectrum the labeled composite particle 35 is discriminated.

In this manner, by discriminating a protein that has specificity in its bonding with the base sequence, a protein like a transcription control factor that can control genes can be discovered, enabling contribution to research into the development of cancers, organisms and the like.

As another application example of the fifth embodiment, an example shown in FIG; 6 (d) will now be described. As shown in FIG. 6 (d), for example, two types of labeled composite particles 35a, 36a are prepared. The target receptors of the labeled composite particle 35a and the labeled composite particle 36a are different, and are formed by DNA fragments with different base sequences. Two types of fluorescent substance 13(○), 15 (△) are bonded with the target receptors in a molar ratio of 4 to 1 for the labeled composite particle 35a, and in a molar ratio of 1 to 4 for the labeled composite particle 36a. In the present embodiment, the micro particles of the two types of labeled composite particles 35a, 36a are formed by magnetic particles which, if an external magnetic field is applied, are magnetized, and if the external magnetic field is removed, are demagnetized. To contact with the magnetic particle 37a on which a DNA binding protein 38, being a selective substance, is fixed, the suspension within which those two types of labeled composite particles 35a, 36a are mixed is dispensed into a container, a column 39 or like that holds the liquid within which the magnetic particle 37a is suspended.

Here, with regard to the magnetic particles which the labeled composite particles 35a, 36a have, the magnetic force received from the same magnetic field is smaller than that for the magnetic particle 37a on which DNA binding protein 38 is fixed. To reduce the magnetic force received, the diameter is either formed smaller with the same material, or a material with low magnetic susceptibility is used. The magnetic body is fitted outside of the liquid path of the pipette device to generate a magnetic field inside the liquid path, or a removable pipette device is used, and hence the operations such as transfer, separation and the like of the labeled composite particles 35a, 36a, and the magnetic particle 37a are simplified and automated.

Furthermore, the magnetic particle 37a receives a stronger magnetic force than the magnetic particles contained in the labeled composite particles 35a, 36a for the same magnetic field. Consequently, in a condition where a predetermined magnetic field is applied; to the container that holds the liquid within which the magnetic particle 37a and the labeled composite particles 35a, 36a are suspended, to the liquid path of the pipette device or to the column 39, labeled composite particles captured by the DNA binding protein 38 of the magnetic particle 37a are adsorbed to the inside wall and separated together with the magnetic particle 37a by passing the suspension through at a predetermined flow rate. However, the labeled composite particles that were not captured by the magnetic particle 37a, whose magnetic force for being adsorbed to the inside wall is weak, flow out with the suspension for selection.

Here, for a micro particle of the labeled composite particle, a nonmagnetic particle may be used. In this case, since the influence of the magnetic field on the labeled composite particle is zero, the labeled composite particles that have not been captured can flow reliably.

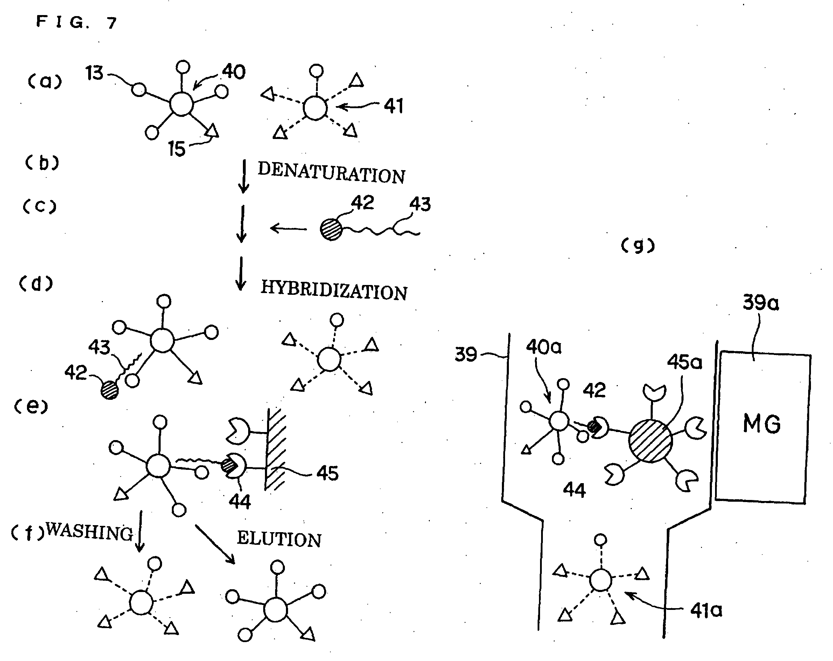

Next is a description of the process for utilizing a labeled composite particle according to a sixth embodiment based on FIG. 7.

As shown in FIG. 7 (a), for example two types of labeled composite particles 40, 41 are prepared. The labeled composite particle 40 and the labeled composite particle 41 each have different target receptors, which are formed by double strand DNA fragments with different base sequences. To label the labeled composite particles 40, 41, the other end of the target receptors are bonded with two types of fluorescent substances 13(○), 15 (△) in a molar ratio of 4 to 1 for the labeled composite particle 40, and in a molar ratio of 1 to 4 for the labeled composite particle 41. In FIG. 7 (b), the target receptors of the two types of labeled composite particles 40, 41 are denatured by an alkaline solution to be a single strand. In FIG. 7 (c), a single strand DNA fragment 43, being a selective substance labeled by DIG42 for testing, is mixed in the suspension within which the aforementioned denatured labeled composite particles 40, 41 are suspended. As shown in FIG. 7 (d), it is tested whether the single strand DNA fragment 43 hybridizes with either one of the labeled composite particles 40, 41. Here, DIG42 is used as a labeled substance having a characteristic that it specifically reacts with anti DIG antibody 44. In FIG. 7 (e), a suspension within which the labeled composite particle 40 with which the single strand DNA fragment 43 has hybridized and the labeled composite particle 41 with which it has not hybridized are mixed, is dispensed into a container that contains a matrix 45 on which the anti DIG antibody 44 is fixed. In FIG. 7 (f), after the labeled composite particles 40, 41 that have not been captured by the matrix 45 are washed for selection, the denatured labeled composite particle 40 is eluted and discriminated by its fluorescence spectrum.

Another application example of the process for utilizing a labeled complex according to a sixth embodiment will now be described based on FIG. 7 (g).

As shown in FIG. 7 (g), for example, two types of labeled composite particles 40a, 41a are prepared. The target receptors of the labeled composite particle 40a and the labeled composite particle 41a are different, and are formed by DNA fragments with different base sequences. Two types of fluorescent substances 13(○), 15 (△) are bonded with the target receptors in a molar ratio of 4 to 1 for the labeled composite particle 40a, and in a molar ratio of 1 to 4 for the labeled composite particle 41a. In the present example, micro particles of the two types of labeled composite particles 40a, 41a are formed by magnetic particles which, if an external magnetic field is applied, are magnetized, and if the external magnetic field is removed, are demagnetized. The target receptors of the two types of labeled composite particles 40a, 41a are denatured into a single strand by an alkaline solution.

A single strand DNA fragment 43 labeled by DIG42 for testing is mixed in the suspension within which the aforementioned denatured labeled composite particles 40a, 41a are suspended, and it is tested whether the single strand DNA fragment 43 hybridizes with either one of the labeled composite particles 40a, 41a. As shown in FIG. 7 (g), the suspension within which the labeled composite particle 40a with which the single strand DNA fragment 43 has hybridized and the labeled composite particle 41a with which it has not hybridized are mixed, is dispensed into a container or a column 39 within which a suspension of magnetic particle 45a on which the anti DIG antibody 44 is fixed is contained. Here, with regard to the magnetic particles which the labeled composite particles 40a, 41a have, the magnetic force received from the same magnetic field is smaller than that of the magnetic particle 45a on which anti DIG antibody 44 is fixed. The magnetic body 39a is fitted outside of the liquid path of the pipette device to generate a magnetic field inside the liquid path, or a removable pipette device is used, and hence the operations such as transfer, separation and the like of the labeled composite particles 40a, 41a, and/or the magnetic particle 45a are simplified and automated.

Furthermore, the magnetic particle 45a receives a stronger magnetic force than the magnetic particles contained in the labeled composite particles 40a, 41a for the same magnetic field. Consequently, in a condition where a predetermined magnetic field is applied; to the container that holds a liquid within which the magnetic particle 45a and the labeled composite particles 40a, 41a are suspended, to the liquid path of the pipette device or to the column 39, labeled composite particles captured by the anti DIG antibody 44 of the magnetic particle 45a are adsorbed to the inside wall and separated together with the magnetic particle 45a by passing the suspension at a predetermined flow rate. However, with regard to the labeled composite particles that were not captured by the magnetic particle 45a, the magnetic force for being adsorbed to the inside wall is weak, so that they flow out with the suspension, and can be selected.

Here, for a micro particle of the labeled composite particle, a nonmagnetic particle may be used. In this case, since the influence of the magnetic field on the labeled composite particle is zero, selection by flowing of labeled composite particles that have not been captured can be performed more reliably.

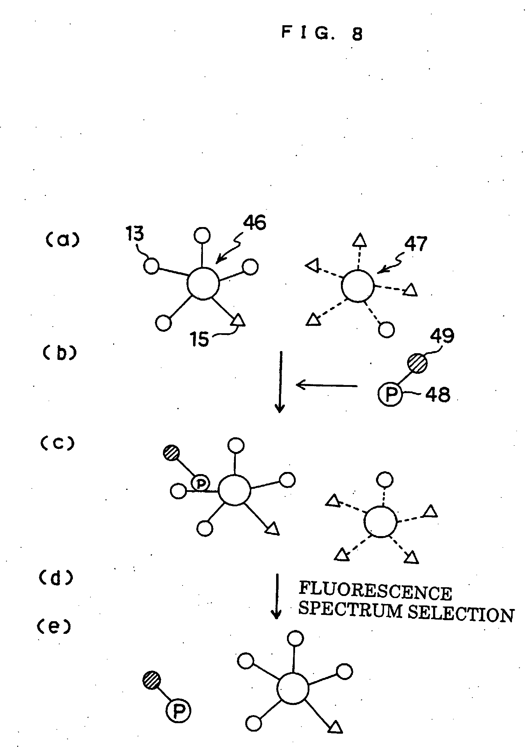

The process for utilizing a labeled composite particle according to a seventh embodiment will now be described based on FIG. 8.

As shown in FIG. 8 (a), for example, two types of labeled composite particles 46, 47 are prepared. The target receptors of the labeled composite particle 46 and the labeled composite particle 47 are different, and are formed by DNA fragments with different base sequences. Two types of fluorescent substances 13(○), 15 (△) are bonded with the target receptors in a molar ratio of 4 to 1 for the labeled composite particle 46, and in a molar ratio of 1 to 4 for the labeled composite particle 47. In FIG. 8 (b), a DNA binding protein 48, being a target labeled by a fluorescent substance 48 is mixed into the suspension within which the two types of labeled composite particles 46, 47 are mixed.

It is assumed, in FIG. 8 (c), that the DNA binding protein 48 is bonded with the labeled composite particle 46. In FIG. 8 (d), by measuring the fluorescence spectrum of the fluorescent substance 48 that is labeled by the DNA binding protein 48, the labeled composite particle 46 by which the DNA binding protein 48 is captured is selected. Then, after the labeled protein 48 is removed in FIG. 8 (e), the labeled composite particle 46 is discriminated by fluorescence.

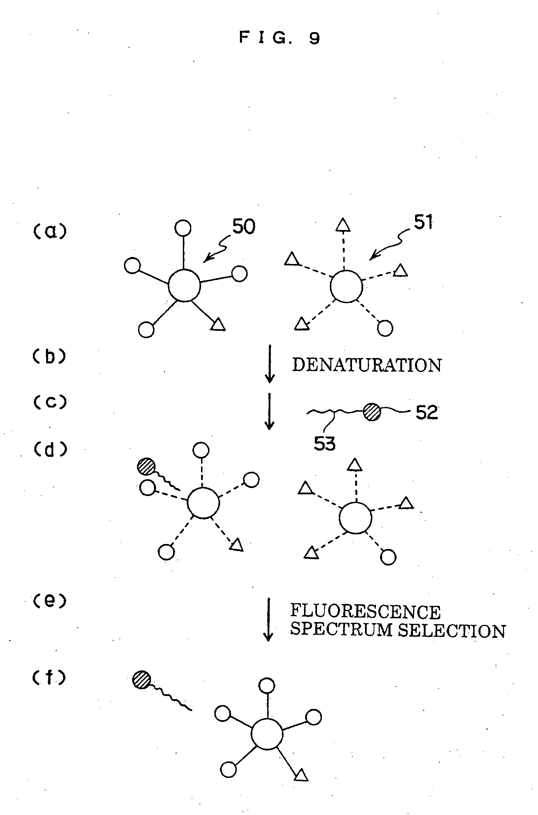

The process for utilizing a labeled composite particle according to an eighth embodiment will now be described based on FIG. 9.

As shown in FIG. 9 (a), for example, two types of labeled composite particles 50, 51 are prepared. One end of each target receptor is fixed onto the labeled composite particles 50, 51. The target receptors of the labeled composite particle 50 and the labeled composite particle 51 are different, and are formed by DNA fragments with different base sequences. Two types of fluorescent substances 13(○), 15 (△) are bonded with the target receptors in a molar ratio of 4 to 1 for the labeled composite particle 50, and in a molar ratio of 1 to 4 for the labeled composite particle 51. In FIG. 9 (b), the two types of labeled composite particles 50, 51 are denatured to be single strands. In FIG. 9 (c), the single strand DNA 53 labeled by the fluorescent substance 52 as a selective substance is mixed into the aforementioned suspension.

In FIG. 9 (d), for example, capture is performed by hybridization with the target receptors of the labeled composite particle 50. In FIG. 9 (e), the labeled composite particle 50 that has captured the labeled single strand DNA is selected by its fluorescence spectrum, and each particle of the labeled composite particles 50, 51 is checked for the existence of the aforementioned fluorescent substance 52 by measuring the fluorescence spectrum. Then, in FIG. 9 (f), after the labeled single strand 53 is removed, the labeled composite particle 50 is discriminated.

As a ninth embodiment, the process for utilizing a labeled complex in order to clearly and efficiently discriminate and observe an influence and/or change that has no clear external indication, will be described..

This is used in the case where the influence by temperature, radiation amount, pressure, weight, magnetic field, electric field, concentration (toxicity, reagent, etc.) electromagnetic wave (visible light, ultraviolet light, X-rays, etc.) particle beam, sound, etc. or combinations of these is not sufficient to appear as an apparent difference, and utilizes a labeled composite particle in order to make the influence explicit and measure it statistically. In the present example, sample groups containing a large number of the same targets such as proteins, DNA fragments, and the like are prepared in advance. Each is subjected to the aforementioned influence, some are subjected to partially changed levels or combinations of them, and some are given none. To discriminate each of these samples, a number of types of suspension of labeled composite particles for the number of the samples are prepared, with which each sample is mixed for the aforementioned targets to hold for labeling. Here, the target receptors of each labeled composite particle are, for example, special adhesive material attached to fibrous material, which can hold various targets.

The labeled composite particles that hold the targets are mixed in the same container, and the same process is performed simultaneously under the same conditions, such as the same time, the same concentration and the like. This process is such that for example, to see the influence on the affinity between a protein and a DNA fragment, a large number of DNA fragments labeled by different types of labeled substances are mixed as selective substances. By analyzing the labeled substances, the aforementioned labeled composite particle that holds the DNA fragment is selected, and by analyzing the labeled substance of the labeled composite particle, the influence that is produced first can be made explicit for rapid and effective measurement. Furthermore, according to the present example, labeling can be performed at a fine level, so that it is possible to measure or detect the critical level and the like that produces a predetermined influence precisely.

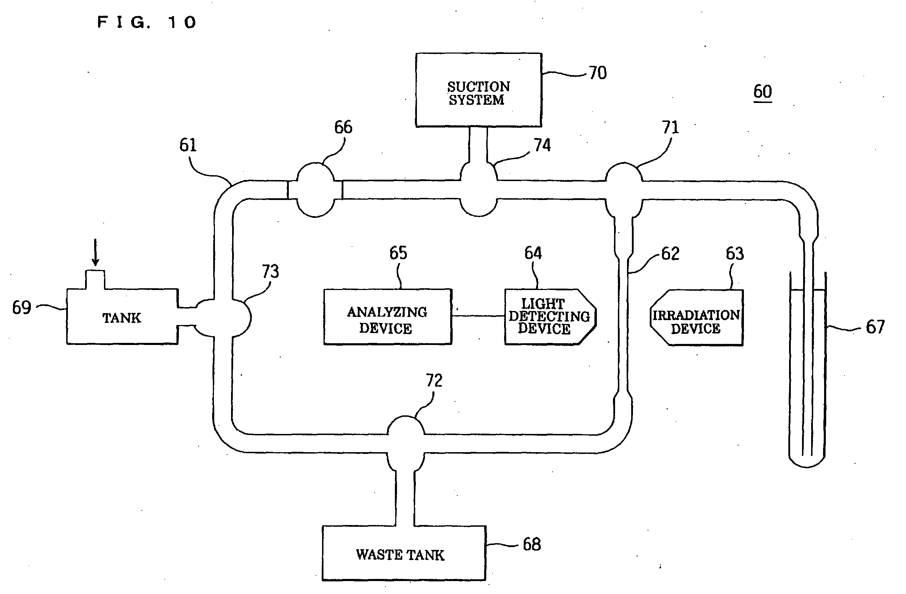

The process for utilizing and the apparatus for utilizing a labeled complex according to a tenth embodiment will now be described based on FIG. 10.

A target analyzer 60, being an apparatus used for the process for utilizing a labeled complex according to the present embodiment is used for the selection of the labeled composite particles and the discrimination of targets. Here, an example in which fluorescent substances or mineral phosphates are used as the labeled composite particles and the labeled substances of selective substances will now be described. The apparatus 60 has a tube (or channel) 61 through which liquid passes, and the tube is formed into a closed circuit such that the liquid can circulate around the inside. A narrow tube 62 formed by a translucent material such as clear glass or the like is provided in a part of the tube 61. An irradiating device 63 that radiates excitation light for exciting the aforementioned fluorescent substances or mineral phosphates is provided outside of the narrow tube 62. Furthermore, a light detecting device 64 is provided outside of the narrow tube 62 on the opposing side of the narrow tube 62 from the irradiating device 63. The irradiating device 63 is provided with a light source such as a laser source, xenon lamp, xenon mercury lamp and the like, with its wavelength selected depending on the aforementioned fluorescent substances and the like. The light detecting device is provided with a photomultiplier tube. Moreover, the arrangement may be such that a filter is used for each device to select the required wavelength. The light detecting device 64 is provided with a measurement device for measuring light wavelength and its intensity.

Moreover, a target analyzer 60 is provided with an analyzing device 65 which analyzes the intensity of the wavelength of light detected by the light detecting device 64, selects the labeled composite particles with which selective substances are bonded, and discriminates targets of the labeled composite particles by the type and molar ratio of the labeled substances of the selected labeled composite particles. The analyzing device 65 is provided with an information processor comprising a CPU, memory, display device and the like, which performs computation for determining the type of fluorescent substance and the like by the wavelength measured, and the molar ratio by the relative light intensity.

At an appropriate location along the tube 61 a transfer pump 66 is provided for pumping liquid through the tube 61. Furthermore, the tube 61 is provided with: a container 67 for holding a suspension of the aforementioned labeled composite particles including the aforementioned selective substances; a waste tank 68 for holding liquid to be disposed of; a tank 69 for holding liquid inside the channel; and a suction system 70 for drawing the suspension held in the container 67 into the tube 61. These elements interconnect via valves 71, 72, 73, and 74, which open and close independently at multiple connecting points. Here, for the suction system 70, a pipette device having a suction and discharge device may be used, or the suction and discharge device may be installed directly. The inner diameter of the tube and the narrow tube are determined depending on the diameter, the flow rate and the like of the labeled composite particles used.

The process for utilizing a labeled complex according to the present embodiment will now be described.

A liquid within which labeled composite particles wherein a fluorescent substance or a mineral phosphate is used as a labeled substance are suspended, and a selective substance labeled by a luminescent substance which is a different type from the labeled substance are mixed. Then, the selective substance is bonded with labeled composite particles to be selected. The suspension of the labeled composite particle group, including the labeled composite particle bonded with the selective substance, is then held in the container 67, the valve 71 is opened, and the suspension is sucked into the tube 61 system by the suction system 70. Next, the valve 71 on the container 67 side is closed to isolate the container 67, and the valve 73 is opened. After the necessary amount of liquid is taken from the tank 69, the valve 73 is closed, and the transfer pump 66 is driven to circulate the suspension inside the tube 61 system. The flow rate is set to a speed at which each labeled composite particle can be measured in the narrow tube 62. Such a flow rate is determined by the inner diameter of the tube and the narrow tube, the diameter of the labeled composite particle, the concentration and the like.

When the suspension passes through the narrow tube 62, excitation light is radiated toward the narrow tube 62 by the irradiating device 63 from outside of the narrow tube 62, and light which labeled composite particles emit from within the narrow tube 62 is detected by the light detecting device 64. The light detecting device 64 can detect light from each particle, so that the analyzing device 65 determines the labeled composite particle with which the selective substance is bonded by measuring the light intensity of each wavelength obtained by the light detecting device 64, and discriminates targets by the type and molar ratio of the labeled substance of the labeled composite particle determined. Here, even if the light detecting device 64 detects light from a plurality of overlapping labeled composite particles, in the present embodiment, since the tube 61 system is closed, by circulating the suspension repeatedly a necessary number of times to obtain sufficient measurement accuracy, such misdetection can be eliminated. After the process is completed, the valve 72 is opened, and the suspension is held in the waste tank 68.

With the present embodiment, selection and discrimination of labeled composite particles can be performed at the same time, and hence the efficiency of the process is good and reliability is high. Furthermore, with the present embodiment, since the suspension can be circulated, measurement can be preformed with high accuracy, and hence reliability is high as expected.

Each of the above-described embodiments is described specifically for the purpose of better understanding the present invention, and does not exclude other embodiments. Accordingly, modification is possible within the scope in which the gist of the invention is not changed. For example, in the above description, only the case of a labeled composite particle having a micro particle as a carrier is described. However, the invention is not limited to this case. For example, substances such as a fibrous material, gel material and the like, or a substance of indeterminate form may be used. Furthermore, two types of fluorescent substances are used as labeled substances. However, the invention is not limited to this case, and many kinds or a mineral phosphate may be used. Moreover, only the case where five pieces of target receptors are fixed to the micro particle is described. However, this is used for simplicity of the description, and to make it easy to describe the molar ratio. In practice a large number of labeled substances are used. Moreover, the above description is only for the case where DNA fragments are used as the target receptors. However, the invention is not limited to this case. Moreover, in the above embodiments, the case is described where nucleic acid is synthesized and amplified by using the PCR method when producing labeled composite particles. However, the invention is not limited to this case and, for example, it may be produced by synthesizing a complementary single strand pair, and forming a double strand by annealing.

## Claims

1. A labeled complex comprising, a carrier, a large number of target receptors bonded with said carrier, and labeled substances bonded with each target receptor at different locations from a location at which said carrier is bonded, wherein said target receptor holds or can hold one or two or more types of targets, and in all of said labeled substances, predetermined types are contained at predetermined molar ratios.

2. A labeled complex according to claim 1, wherein all of said labeled substances are distributed to almost all target receptors bonded with one carrier, and one target receptor is bonded with one type of labeled substance.

3. A labeled complex according to either one of claim 1 and claim 2, wherein said target receptor, which is bonded with the carrier on a part thereof, and bonded with the labeled substance on the other part thereof, is formed in a slender shape such as a line, a thread, a hair, a stick and the like.

4. A labeled complex according to any one of claim 1 through claim 3, wherein said target receptors comprise chemical compounds which contain biopolymers such as nucleic acids, peptides, proteins, polysaccharides, lipids and the like, or living beings such as viruses, bacteria and the like or a part thereof, or substances which hold or are able to hold them.

5. A labeled complex according to any one of claim 1 through claim 4, wherein said target receptors comprise nucleic acids having a predetermined double strand base sequence, said labeled substance is bonded with only a single strand at one location, and said carrier is bonded with the other single strand in at another location.

6. A labeled complex according to any one of claim 1 through claim 4, wherein said target receptor comprises nucleic acid having a predetermined double strand base sequence, said labeled substance is bonded only with one location of a single strand, and said carrier is fixed to another location of the single strand.

7. A labeled complex according to any one of claim 1 through claim 4, wherein said target receptor is a single strand nucleic acid.

8. A labeled complex according to any one of claim 1 through claim 6, wherein said labeled substance is a fluorescent substance, mineral phosphate, or luminescent substance of a chemiluminescent substance and the like;

9. A labeled complex according to claim 8, wherein the type of said luminescent substance can be discriminated by any one of the excitation wavelength, emission wavelength, emission intensity, degree of emission polarization, emission phase or emission lifetime.

10. A labeled complex according to claim 9, wherein said carrier is coated with one of a pair of chemical compounds that are specifically bonded, such as avidin, biotin and the like, said target receptor is a DNA fragment having a predetermined base sequence, the other chemical compound of said chemical compound pair is bonded at one position, and said fluorescent substance is bonded at an other position.

11. A labeled complex according to any one of claim 1 through claim 9, wherein said carrier has objects of action at a distance such as magnetic particles and the like, which can be controlled remotely.

12. A process for producing a labeled complex according to any one of claim 1 through claim 11, said process having; a step for forming target receptors, which are bonded with labeled substances at one place, and hold or are capable of holding specific targets, and a step for bonding the target receptors with the carrier.

13. A process for producing a labeled complex according to claim 12, wherein said step for bonding target receptors with said carriers is performed by mixing the carriers with which the target receptors are to be bonded, in a liquid wherein a large number of target receptors which are bonded with labeled substances are suspended such that all of the labeled substances are of the types and molar ratios that are determined according to the types of the target receptors or the types and the quantity ratios of the target receptors.

14. A process for producing a labeled complex according to claim 12, wherein said step for generating the target receptors has a step for synthesizing a single strand nucleic acid that is bonded with a labeled substance, and that has a predetermined base sequence, and synthesizing another single strand nucleic acid that has a high relation with the base sequence, and that is processed to be capable of being bonded with the carriers, and generating a double strand nucleic acid by annealing these.

15. A process for producing a labeled complex according to claim 12, wherein said step for generating the target receptors has a step wherein, by using a first primer for reproduction of a single strand nucleic acid that is bonded with said labeled substances and that has a predetermined base sequence, and a second primer for reproduction of the other single strand nucleic acid to be bonded with said carrier, a double strand nucleic acid is synthesized and amplified.

16. A process for producing a labeled complex according to claim 12, wherein said step for bonding the target receptors with carriers, or the step for generating the target receptors, synthesizes and amplifies double strand nucleic acid by using a first primer for reproduction of a single strand nucleic acid with a predetermined base sequence, which combines with either one of said labeled substance and said carrier, and also provides a restriction enzyme process at the opposite end to the end that is bonded with said labeled substance or a carrier and, via an adapter composed of DNA ligase and the like, bonds a carrier or a labeled substance onto the single strand side to combine the target receptor with the carrier, or generates a target reservoir.

17. A process for producing a labeled complex according to claim 16, wherein said step for generating the target receptor includes a step for removing a single strand that is not bonded with said labeled substance or said carrier by denaturation.

18. A process for producing a labeled complex according to claim 16, wherein said step for bonding the target receptor with the carrier bonds said target receptor and said carrier by utilizing bonding including physical or chemical bonding such as attachment, adsorption, adhesion through the many holes, gaps, or irregularities that the carrier has, or a specific interaction of biotin, avidin and the like, to suspend said target receptor and said carrier.

19. A process for producing a labeled complex according to claim 16, wherein said step for generating the target receptor is a step for generating a plurality of target receptors with which is bonded one of a pair of chemical compounds, one part of which is bonded with the labeled substance, and the other part of which is specifically bonded, and said step for bonding the target receptor with the carrier is a step for bonding the target receptor with the carrier by suspending in liquid the carrier on which the other of said pair of chemical compounds is coated, and said, target receptor with which the labeled substance is bonded.

20. A process for utilizing a labeled complex having; a step for selecting a labeled complex whose type is targeted from among the labeled complex group having a large number of a plurality of types of labeled complexes wherein the types or molar ratio of the targets of the labeled complex according to any one of claim 1 through claim 11, and the labeled substances assigned to the targets are different from each other, and a step wherein the selected labeled complex discriminates the labeled target.

21. A process for utilizing a labeled complex according to claim 20, wherein said selection step has a liquid path and magnetic means which is capable of applying magnetization to inside of the liquid path, and is performed for the labeled complex, or the labeled complex and selective substances, using a pipette device for suction and discharge, and by operations such as quantification, isolation, apportioning, dispensing, clarity, suspension, agitation, concentration, dilution, mixing, contact, capture, holding, washing, denaturation, incubation, temperature control, extraction, recovery, transport, and the like, or by a combination of these operations.

22. A process for utilizing a labeled complex according to either one of claim 20 and claim 22, wherein said selection step has; a step for suspending said labeled complex group, a step for contacting the suspension in which the labeled complex group is suspended, and selective substances for selecting the object labeled complexes, and a step for extracting or separating the labeled complexes bonded with the selective substances.

23. A process for utilizing a labeled complex according to claim 22, wherein said selection step has a step for labeling said selective substances with different types of labeled substances from the labeled substances contained in the labeled complex, and for extracting and separating the labeled complex bonded with the selective substances based on the labeled substances.

24. A process for utilizing a labeled complex according to either one of claim 20 and claim 21, wherein said discrimination step, in the case where said labeled substances are luminescent substances, computes the ratio of the amount of the type based on a result of the measurement of intensity of each emission wavelength which the labeled complex emits, to discriminate the corresponding target.

25. A process for utilizing a labeled complex according to either one of claim 20 and claim 21, wherein with said selection step, said target receptor is a double strand nucleic acid with a predetermined base sequence, and in the case where said labeled substance and said carrier are bonded with only one single strand thereof, the other single strand is removed by denaturation, and moreover for said selective substances, nucleic acid having a predetermined base sequence is used.

26. A process for utilizing a labeled complex according to either one of claim 20 and claim 21, wherein said selection step has a step for contacting stationary phases on which selective substances are fixed, and a liquid wherein the labeled complex group is suspended, and a step for selecting a labeled complex bonded with the selective substances on the stationary phase by removing the suspension by washing.

27. A process for utilizing a labeled complex according to claim 26, wherein said selection step has a step wherein labeled complexes are eluted physically or chemically from said stationary phases for extraction, and are selected by separation, washing or the like.

28. A process for utilizing a labeled complex according to claim 26, wherein magnetic particles are used for said stationary phases, the carriers of said labeled complex are formed by magnetic particles or non-magnetic particles, and compared with the magnetic particles of the stationary phase, the magnetic particles of the labeled complex experience a smaller magnetic field for the same external magnetic field.

29. A process for utilizing a labeled complex according to claim 26, wherein said selection substance is labeled with a labeled substance of one of a pair of chemical compounds that are specifically bonded, and said selection step has a step where a liquid wherein the conjugation of the selective substances and labeled complexes is suspended, is contacted with the stationary phase on which the other of said pair of chemical compounds is fixed, the conjugation is bonded with said stationary phase, the substances other than those bonded with the stationary phase are washed to be removed, said target receptor is denatured to be a single strand, and the labeled complex is eluted and selected by extraction, or separation.

30. A process for utilizing a labeled complex according to either one of claim 20 and claim 21, wherein said selection step has; a step for labeling said labeled complex by luminescent substances, for labeling the selective substances by different types of luminescent substances from the labeled substances, and for mixing and contacting the liquid in which the labeled complex group is suspended with the selective substances, and a step for passing suspended liquid of the labeled complex group including labeled complexes bonded with the selective substances through a translucent narrow tube, and said discrimination step has a step for receiving light when the suspended liquid of said labeled complex group passes through said narrow tube, and a step wherein, with respect to the labeled complex selected by the measurement of the intensity of light emitted by the selective substance, based on the result of a measurement of the intensity of light emitted by the labeled complex, the types and the molar ratio are computed to discriminate the corresponding target.

31. A process for utilizing a labeled complex according to either one of claim 21, wherein in the case where said discrimination substances or selective substances are fluorescent substances or mineral phosphates, in the step for passing said suspended liquid through said narrow tube, an excitation light for exciting the substances is emitted toward said narrow tube.

32. A target analyzing apparatus which utilizes a labeled complex, said apparatus having a transfer pump for transferring liquid within which a labeled complex group is suspended, a translucent narrow tube through which the suspension passes, light detecting means for detecting light from the discrimination substances and selective substances of the labeled complex when passing through the narrow tube, and analyzing means for analyzing the intensity of light received by said light detecting means, selecting the labeled complex, and discriminating a target.

33. A target analyzing apparatus which utilizes a labeled complex according to claim 32, wherein there is further provided irradiating means for externally radiating excitation light toward said narrow tube for, in the case where said discrimination substances or selective substances are fluorescent substances or mineral phosphates, exciting the substances, or providing light for scattering to obtain scattered light from the substances.

34. A target analyzing apparatus which utilizes a labeled complex according to claim 32, wherein said narrow tube forms part of a closed circuit, and the suspension of said labeled complex group is circulated in the closed circuit.
